# EUROPEAN PATENT APPLICATION

(11) **EP 4 382 035 A1**
(43) Date of publication of application: **12.06.2024**
(21) Application number: 22852795.8
(22) Date of filing: 12.07.2022
(51) Int. Cl.: A61B 3/15

(54) **OPHTHALMIC DEVICE**

(30) Priority: 02.08.2021 JP 2021126964
(71) Applicant: TOPCON CORPORATION, Tokyo 174-8580 (JP)
(72) Inventor: OKI Takuya, Tokyo 174-8580 (JP); YAMAOKA Masashi, Tokyo 174-8580 (JP)
(74) Representative: Louis Pöhlau Lohrentz
(86) International application number: PCT/JP2022/027425
(87) International publication number: WO 2023/013369

(57) **Abstract**

To provide an ophthalmic device in which an operator can objectively grasp a positional relationship between an eye to be examined and am acquisition optical system, and an operability when the operator controls a position or an orientation of the acquisition optical system can be improved, an ophthalmic device includes an acquisition optical system (20) that acquires eye information of an eye (E) to be examined; an electric position change mechanism (15) that changes a position and .an angle change mechanism (17) that changes an orientation of the acquisition optical system (20) with respect to the eye (E) to be examined; a display (25) that is visually recognizable by an operator who operates the electric position change mechanism (15) and the angle change mechanism (17); and a controller (30) that displays, on the display (25), positional relationship information (J) indicating a positional relationship between the eye (E) to be examined and the acquisition optical system (20).

## Description

### TECHNICAL FIELD

The present invention relates to an ophthalmic device.

### BACKGROUND ART

Conventionally, an ophthalmic device including an acquisition optical system that acquires eye information of an eye to be examined is known. In a conventional ophthalmic device, an operator such as an examiner performs a touch operation on an observation image of an eye to be examined displayed on a display having a touch panel function, and the acquisition optical system is moved based on the touch operation (see, for example, Patent Literature 1).

### CITATION LIST

### Patent Literature

Patent Literature 1: JP6815722B

### SUMMARY

### Technical Problem

By the way, in the conventional ophthalmic device, it is necessary to estimate a positional relationship between the eye to be examined and the acquisition optical system based on the observation image of the eye to be examined displayed on the display. That is, when moving the acquisition optical system, the operator needs to move the acquisition optical system based on a subjective positional relationship estimated from the observation image. Therefore, when the positional relationship between the eye to be examined and the acquisition optical system estimated by the operator is different from the actual positional relationship, the acquisition optical system cannot be moved to a desired position. That is, in the conventional ophthalmic device, the operator cannot objectively grasp the positional relationship between the eye to be examined and the acquisition optical system, and there is a problem that operability is low when the operator controls a position or an orientation of the acquisition optical system.

The present invention has been made in view of the above problems, and an object of the present invention is to provide an ophthalmic device capable of allowing an operator to objectively grasp a positional relationship between an eye to be examined and an acquisition optical system and improving operability when the operator controls a position or an orientation of the acquisition optical system.

### Solution to Problem

In order to achieve the above object, an ophthalmic device of the present invention includes an acquisition optical system that acquires eye information of an eye to be examined, an electric optical system change mechanism that changes at least one of a position or an orientation of the acquisition optical system with respect to the eye to be examined, a display that is visually recognizable by an examiner who operates the optical system change mechanism, and a controller that displays, on the display, positional relationship information indicating a positional relationship between the eye to be examined and the acquisition optical system.

### Advantageous Effects

Therefore, in the ophthalmic device of the present invention, the operator can obj ectively grasp the positional relationship between the eye to be examined and the acquisition optical system, and the operability when the operator controls a position or an orientation of the acquisition optical system with respect to the eye to be examined can be improved.

### BRIEF DESCRIPTION OF DRAWINGS

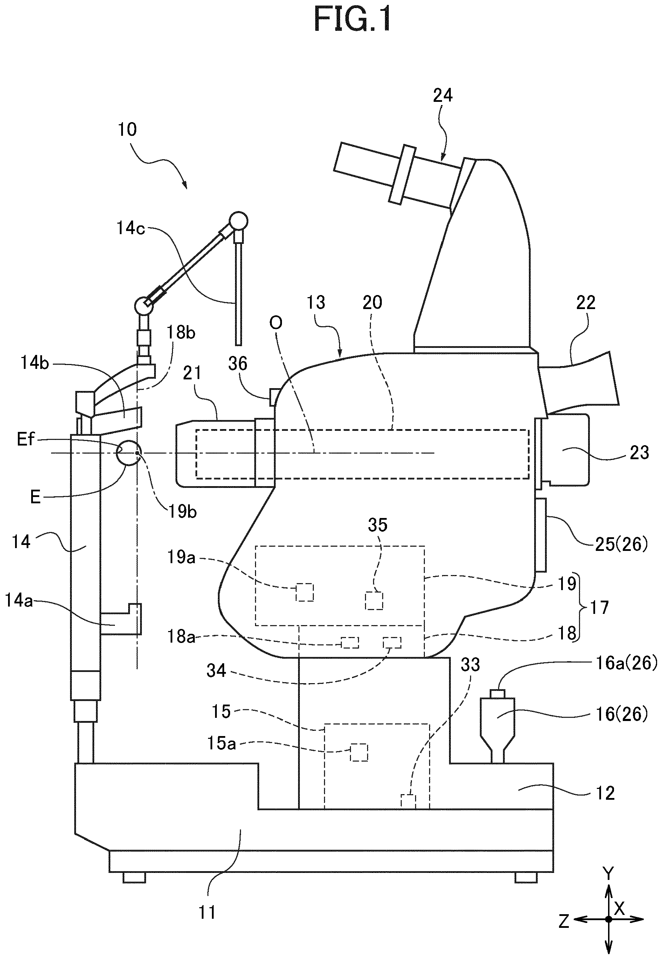

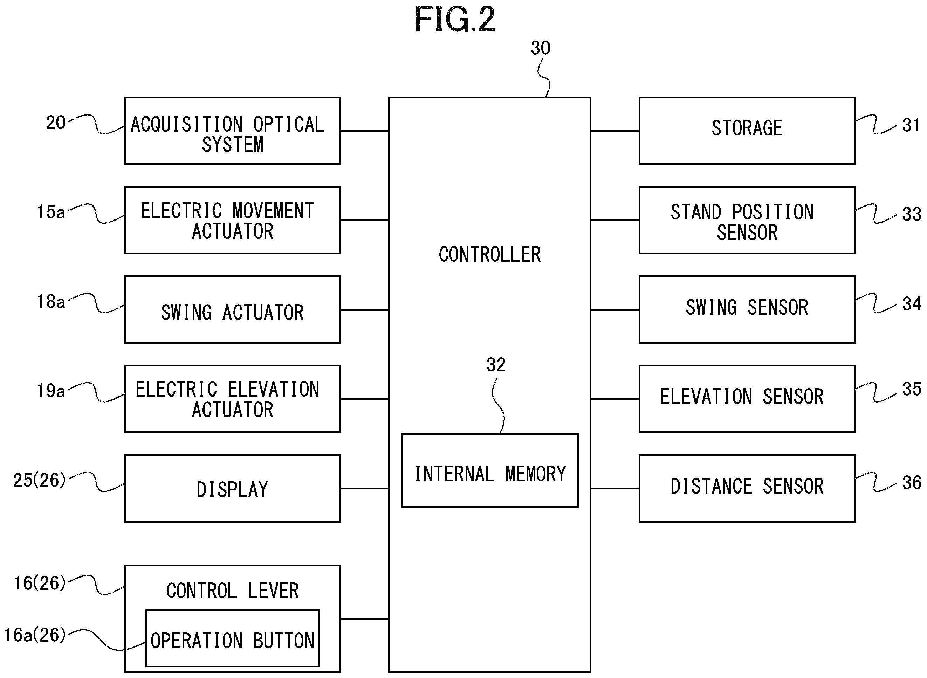

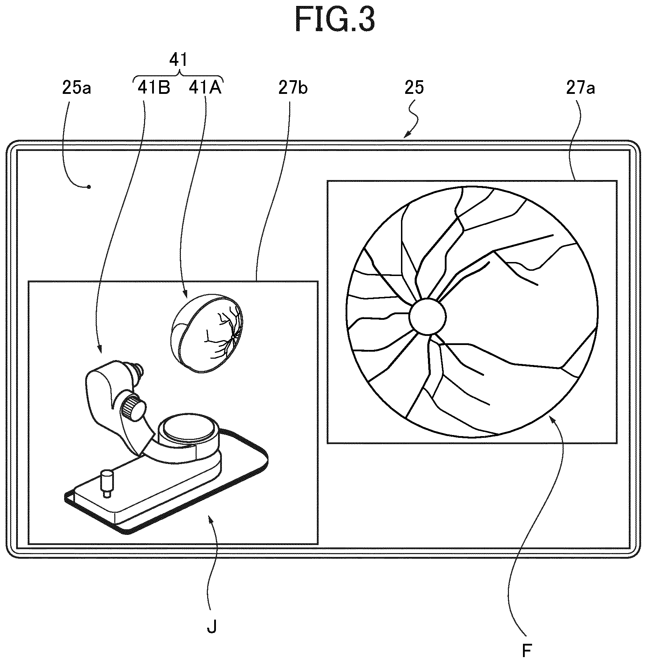

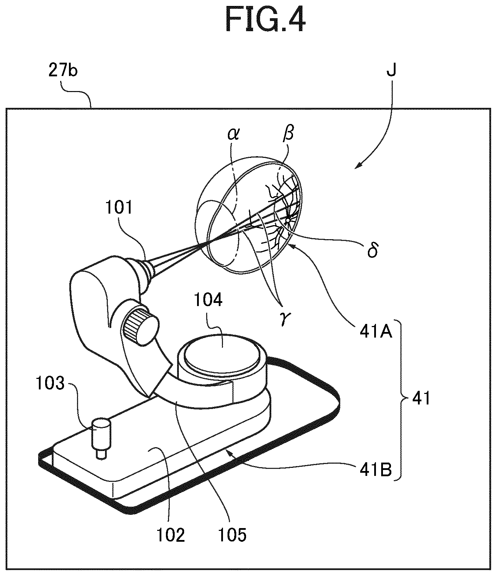

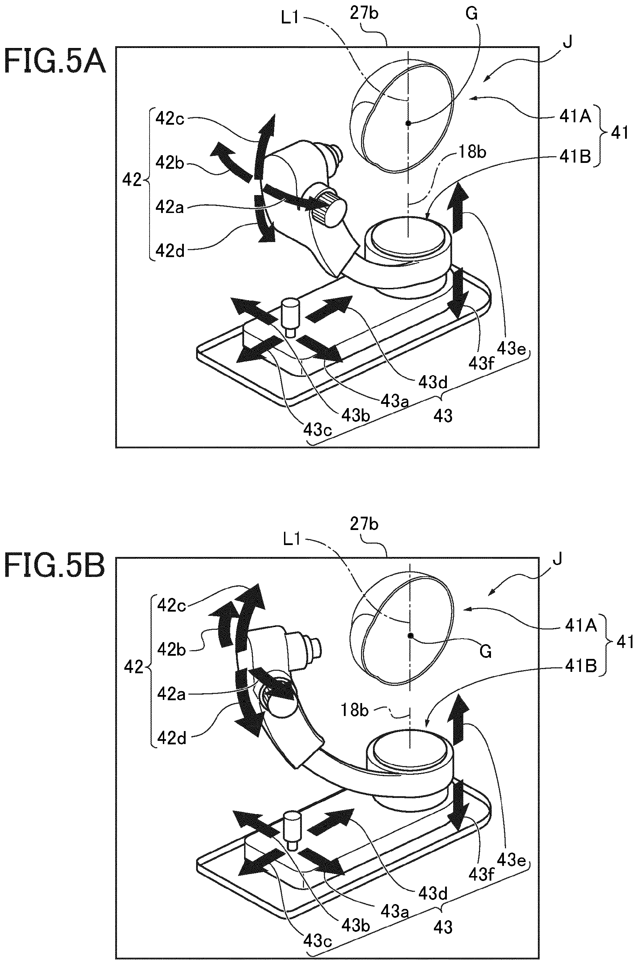

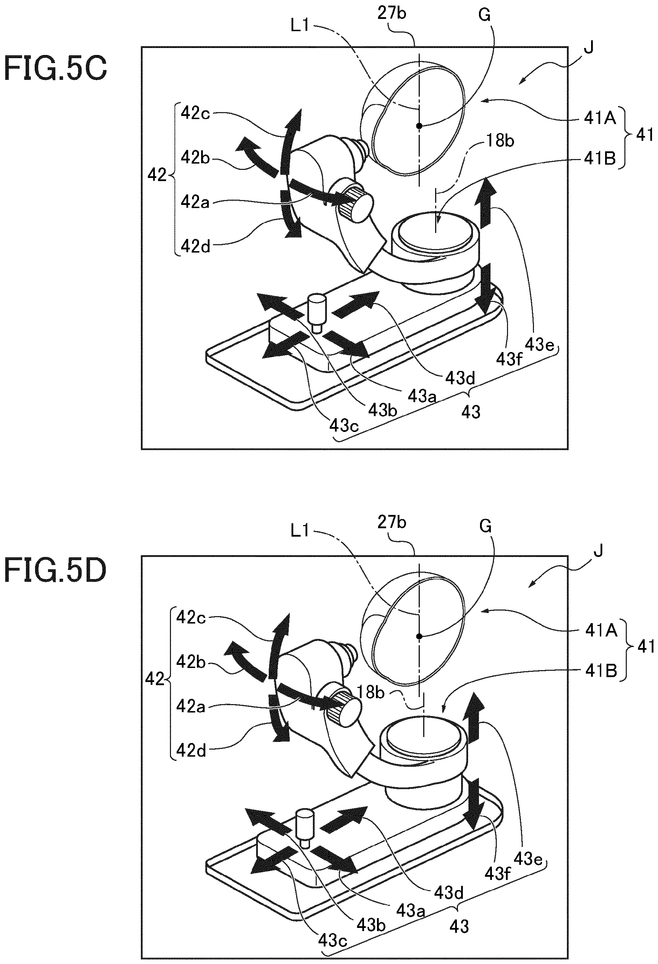

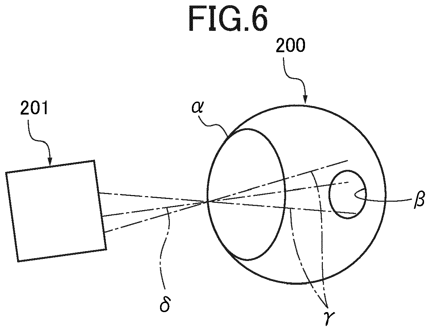

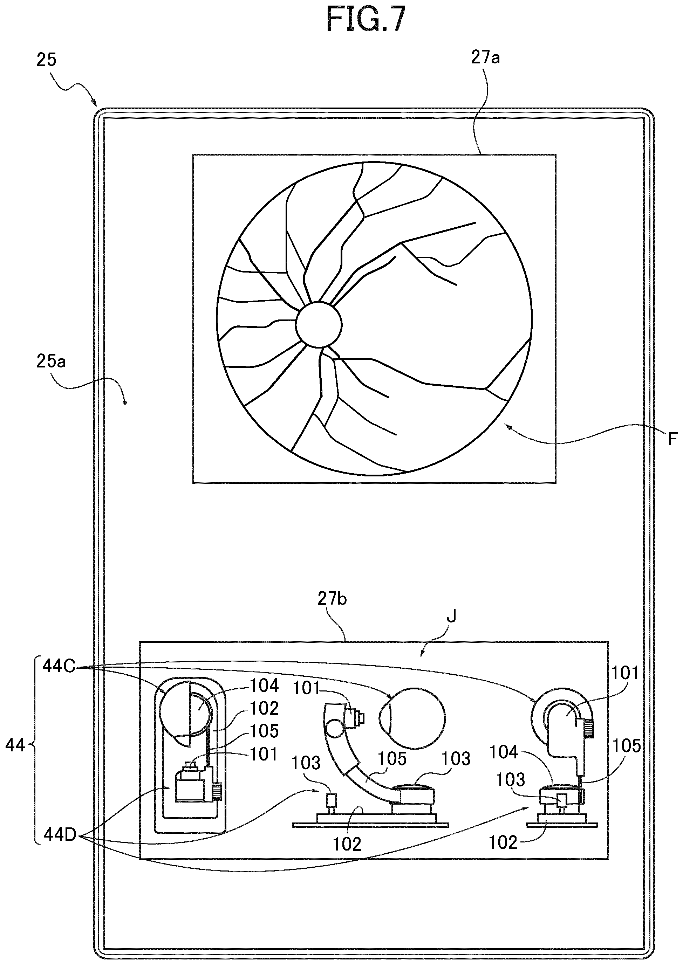

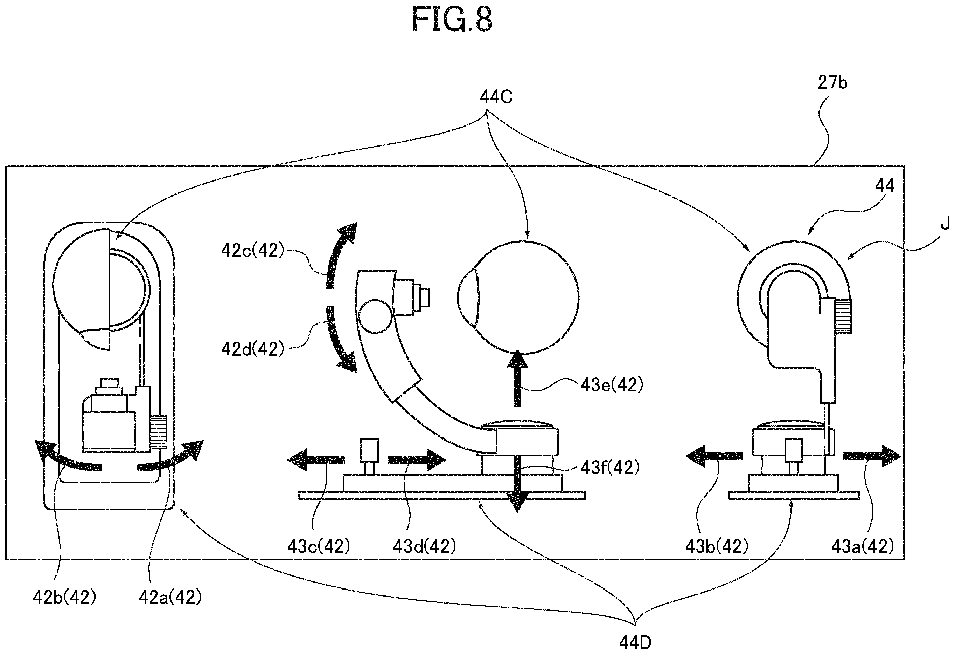

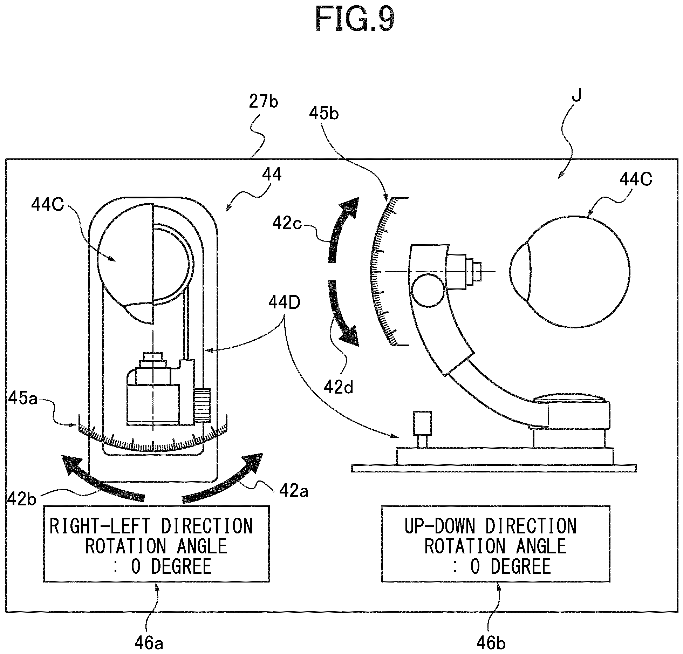

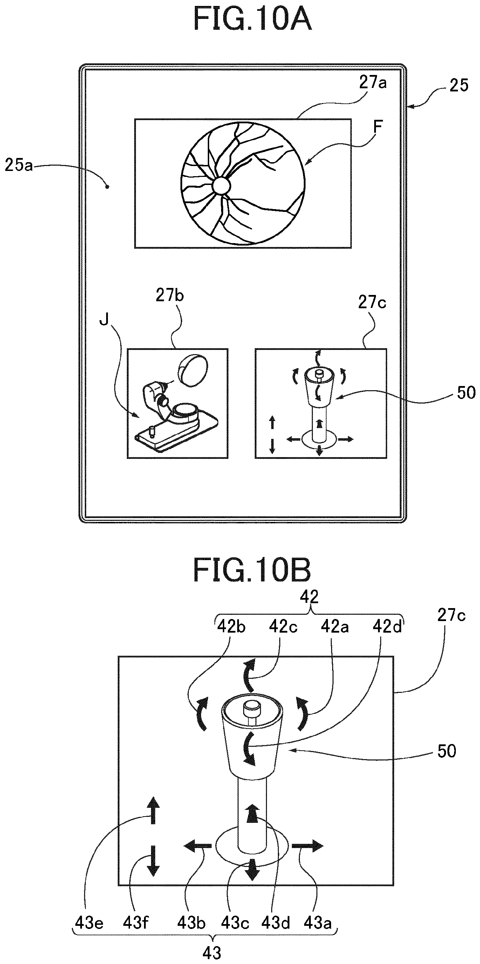

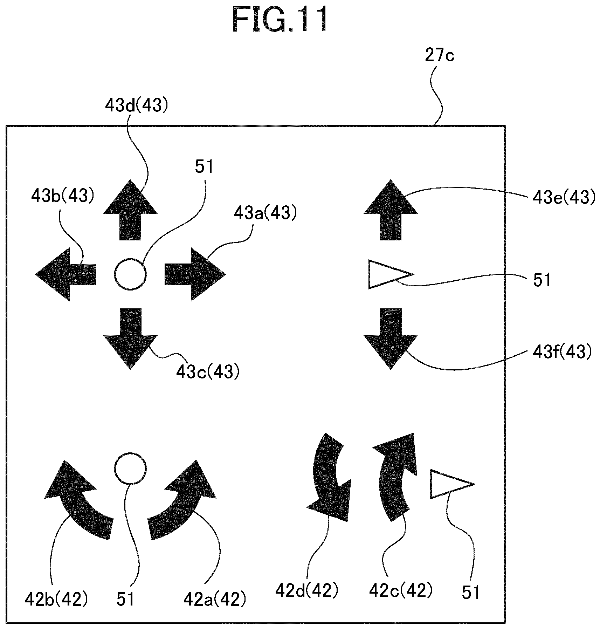

Fig. 1 is an overall schematic view illustrating a configuration of an ophthalmic device of a first embodiment. Fig. 2 is a block view illustrating a control configuration of the ophthalmic device of the first embodiment. Fig. 3 is an explanatory view illustrating a display example of a display of the ophthalmic device of the first embodiment. Fig. 4 is an explanatory view illustrating positional relationship information displayed by the ophthalmic device of the first embodiment. Fig. 5A is an explanatory view in which software keys are superimposed and displayed on positional relationship information according to the first embodiment. Fig. 5B is a first explanatory view in which display of the positional relationship information is changed according to an actual orientation of an acquisition optical system. Fig. 5C is a second explanatory view in which the display of the positional relationship information is changed according to an actual position of the acquisition optical system. Fig. 5D is a third explanatory view in which the display of the positional relationship information is changed according to the actual position of the acquisition optical system. Fig. 6 is an explanatory view illustrating a modification of a light flux image and an optical axis image displayed as the positional relationship information. Fig. 7 is an explanatory view illustrating a display example of a display of an ophthalmic device of a second embodiment. Fig. 8 is an explanatory view illustrating positional relationship information displayed by the ophthalmic device of the second embodiment. Fig. 9 is an explanatory view illustrating a modification of positional relationship information in the ophthalmic device of the second embodiment. Fig. 10A is an explanatory view illustrating a modification of display on the display of the ophthalmic device of the first embodiment. Fig. 10B is an explanatory view illustrating enlarged display of a third display area. Fig. 11 is an explanatory view illustrating a modification of display of the third display area illustrated in Fig. 10B.

### DESCRIPTION OF EMBODIMENTS

Embodiments for carrying out the ophthalmic device of the present invention will be described based on a first embodiment and a second embodiment illustrated in the drawings. In the following description, when viewed from a side facing a subject (examiner side), a left-right direction is indicated by an arrow X, an up-down direction (vertical direction) is indicated by an arrow Y, and a direction orthogonal to the left-right direction and the up-down direction is indicated by an arrow Z as a front-rear direction. In the left-right direction (X-axis direction), a left side of the examiner is defined as a left direction, and a right side of the examiner is defined as a right direction. Furthermore, in the front-rear direction (Z-axis direction), the examiner side is defined as a front side, and the subject side is defined as a rear side.

### (First Embodiment)

Hereinafter, a configuration of an ophthalmic device 10 of the first embodiment will be described with reference to Figs. 1 and 2.

The ophthalmic device 10 of the first embodiment is an eye fundus camera, and has an acquisition optical system 20 that acquires an eye fundus image as eye information of an eye E to be examined.

As illustrated in Fig. 1, the ophthalmic device 10 has a base 11, a stand 12, a main body portion 13, and a support column 14. The acquisition optical system 20 is accommodated in the main body portion 13.

The stand 12 is installed on the base 11 and supported by the base 11 via a position change mechanism 15. The position change mechanism 15 is a known electric moving mechanism that moves the stand 12 in the left-right direction (X-axis direction), the front-rear direction (Z-axis direction), and the up-down direction (Y-axis direction), respectively. That is, the position change mechanism 15 has an electric movement actuator 15a such as a stepping motor that generates a driving force for moving the stand 12, and a transmission mechanism (not illustrated) that transmits the driving force generated by the movement actuator 15a to the stand 12. Then, the position change mechanism 15 moves the stand 12 by using the driving force of the movement actuator 15a. Note that the movement actuator 15a may be provided individually for one that moves the stand 12 in the left-right direction, one that moves the stand 12 in the front-rear direction, and one that moves the stand 12 in the up-down direction, or may also be used for one that moves the stand 12 in a plurality of directions.

The stand 12 is provided with a control lever 16 which is an operation portion 26 described later. When the control lever 16 is operated by the examiner who is the operator, the controller 30 described later outputs a control command to the movement actuator 15a of the position change mechanism 15 or a swing actuator 18a or an elevation actuator 19a of an angle change mechanism 17 described later according to the operation. An operation button 16a is disposed at a distal end portion of the control lever 16. When the operation button 16a is pressed by the examiner, the controller 30 outputs a control command for imaging an eye fundus image of an eye E to be examined to the acquisition optical system 20.

The main body portion 13 is installed on the stand 12 and supported by the stand 12 via the angle change mechanism 17. Here, the main body portion 13 accommodates the acquisition optical system 20 and the controller 30 (see Fig. 2). In addition, the main body portion 13 is provided with an objective lens portion 21 and an eyepiece lens portion 22. Further, a still camera 23 and an imaging device 24 are detachably coupled to the main body portion 13.

The angle change mechanism 17 is a known electric moving mechanism that rotates the main body portion 13 in the left-right direction and the up-down direction. The angle change mechanism 17 has a swing mechanism (swing mechanism) 18 and an elevation mechanism (tilt mechanism) 19.

The swing mechanism 18 has an electric swing actuator 18a such as a stepping motor that generates a driving force for rotating the main body portion 13, and a transmission mechanism (not illustrated) that transmits the driving force generated by the swing actuator 18a to the main body portion 13. The swing mechanism 18 uses the driving force of the swing actuator 18a to rotate the main body portion 13 in the left-right direction (X-axis direction) about a preset rotation axis 18b (reference axis). Here, the rotation axis 18b is set as a straight line passing through a predetermined position set on the rear side of the acquisition optical system 20 in the front-rear direction (Z-axis direction) and extending in the up-down direction (Y-axis direction). The acquisition optical system 20 is aligned such that the rotation axis 18b coincides with a pupil center position of the eye E to be examined. In the state illustrated in Fig. 1, the pupil center position of the eye E to be examined coincides with the rotation axis 18b.

The elevation mechanism 19 has an electric elevation actuator 19a such as a stepping motor that generates a driving force for rotating the main body portion 13, and a transmission mechanism (not illustrated) that transmits the driving force generated by the elevation actuator 19a to the main body portion 13. The elevation mechanism 19 rotates the main body portion 13 in the up-down direction (Y-axis direction) about a preset center axis 19b (reference axis) by using the driving force of the elevation actuator 19a. Here, the center axis 19b is set as a straight line passing through a position where the rotation axis 18b intersects an optical axis O of the acquisition optical system 20 and extending in the left-right direction (X-axis direction).

In the ophthalmic device 10 of the first embodiment, the position change mechanism 15 automatically moves the stand 12 in each of the left-right direction, the front-rear direction, and the up-down direction, and the angle change mechanism 17 automatically rotates the main body portion 13 installed on the stand 12 in each of the left-right direction and the up-down direction. That is, in the acquisition optical system 20 accommodated in the main body portion 13, the position in the left-right direction, the front-rear direction, and the up-down direction is changed together with the stand 12 by the position change mechanism 15, and the rotation angle (orientation) in the left-right direction and the up-down direction is changed together with the main body portion 13 by the angle change mechanism 17. Therefore, the position change mechanism 15 and the angle change mechanism 17 correspond to an electric optical system change mechanism that changes a position and an orientation of the acquisition optical system 20 with respect to the eye E to be examined.

The support column 14 stands upright from the base 11 and extends in the up-down direction. The support column 14 is provided with a chin receiving portion 14a, a forehead-applied portion 14b, and an external fixation lamp 14c. When acquiring the eye information of the eye E to be examined, the chin receiving portion 14a and the forehead-applied portion 14b fix the face of the subject (patient), that is, the position of the eye E to be examined with respect to the main body portion 13 (acquisition optical system 20). The chin receiving portion 14a is a place where the subject places his/her chin, and the forehead-applied portion 14b is a place where the subject puts his/her forehead. The chin receiving portion 14a and the forehead-applied portion 14b are movable in the up-down direction with respect to the base 11 respectively. The external fixation lamp 14c is a light source that fixes the eye E to be examined (fixes the line of sight). In the ophthalmic device 10 of the first embodiment, the subject places his/her chin on the chin receiving portion 14a and puts his/her forehead on the forehead-applied portion 14b, and the external fixation lamp 14c is appropriately turned on in a state of facing the main body portion 13 to perform inspection, observation, photographing, and the like of the eye E to be examined.

The acquisition optical system 20 accommodated in the main body portion 13 is an optical system that acquires eye information (eye fundus image) of the eye E to be examined. The acquisition optical system 20 has an illumination optical system that illuminates an eye fundus Ef, a photographing optical system that observes and photographs the illuminated eye fundus Ef, an objective lens, an eyepiece lens, and the like.

The illumination optical system forms a ring light-transmitting portion image of the observation illumination light on the pupil of the eye E to be examined in order to use the eye fundus reflection image from the eye E to be examined at the time of eye fundus observation. In addition, the illumination optical system illuminates the eye fundus Ef by causing the xenon lamp to emit flash light at the time of photographing the eye fundus. In the case of fluorescence photographing, the illumination optical system can switch an exciter filter according to FAG photographing or ICG photographing. Further, the illumination optical system retracts the exciter filter from the optical path in a case of color photographing.

The photographing optical system guides reflected light from the eye E to be examined illuminated by the illumination optical system to a photographing medium of the still camera 23 or an imaging element of the imaging device 24, and enables observation and photographing of the eye fundus Ef.

The objective lens portion 21 is configured by an objective lens (not illustrated) of the acquisition optical system 20 accommodated in a lens barrel. The objective lens portion 21 is disposed at a position facing the eye E to be examined. The eyepiece lens portion 22 is configured by an eyepiece lens (not illustrated) of the acquisition optical system 20 accommodated in the lens barrel. The eyepiece lens portion 22 is a portion where the examiner observes the eye E to be examined.

The still camera 23 photographs a still image of the eye fundus Ef of the eye E to be examined through the acquisition optical system 20. As the still camera 23, a digital camera equipped with a charge coupled device (CCD), a film camera, an instant camera, or the like is used according to the purpose of inspection or the like. The imaging device 24 photographs a moving image or the like of the eye fundus Ef of the eye E to be examined through the acquisition optical system 20. As the imaging device 24, a television camera or the like is used. Note that in a case where the still camera 23 or the imaging device 24 are of a digital imaging method, image data acquired by a recording medium in the ophthalmic device 10, an image recording device such as a computer provided outside, or the like can be saved.

Furthermore, the main body portion 13 is provided with a display 25. The display 25 is disposed at a position facing the examiner during the acquisition of the eye information. The display 25 is a display device that can be visually recognized by the examiner who is the operator operating the position change mechanism 15 and the angle change mechanism 17. The display 25 includes a liquid crystal display device having a touch panel function on a display screen 25a (see Fig. 3).

On the display screen 25a of the display 25, an image of the eye fundus Ef of the eye E to be examined based on the image data from the acquisition optical system 20, a software key as the operation portion 26, and the like are appropriately displayed under the control of the controller 30. Further, positional relationship information J to be described later is displayed on the display screen 25a.

The operation portion 26 is used by the examiner or the subject to operate, set, and the like of the chin receiving portion 14a, the forehead-applied portion 14b, the position change mechanism 15, the angle change mechanism 17, the acquisition optical system 20, and the like. The operation portion 26 is operated by the examiner or the like to output a predetermined operation signal corresponding to the operation to the controller 30. The controller 30 outputs a predetermined control command to the chin receiving portion 14a, the forehead-applied portion 14b, the position change mechanism 15, the angle change mechanism 17, the acquisition optical system 20, and the like based on the operation signal from the operation portion 26. As a result, the operation, setting, and the like of the chin receiving portion 14a and the like are operated.

The operation portion 26 includes a control lever 16, an operation button 16a provided at a distal end portion of the control lever 16, a software key displayed on the display 25, and the like. The software key enables various operations such as alignment of the acquisition optical system 20, setting of various inspection conditions, and adjustment of display contents of the display 25. Note that the operation portion 26 may also have various buttons provided around the control lever 16 and around the display 25. Furthermore, the operation portion 26 may include an input device such as a keyboard and a mouse.

As illustrated in Fig. 2, the controller 30 develops a program stored in a storage 31 or an internal memory 32 built in on, for example, a random access memory (RAM), thereby comprehensively controlling the operation of the ophthalmic device 10 according to the operation or the like on the operation portion 26. In the first embodiment, the internal memory 32 includes a RAM or the like, and the storage 31 includes a read only memory (ROM), an electrically erasable programmable ROM (EEPROM), or the like.

In addition to the above-described configuration, the ophthalmic device 10 may be appropriately provided with a printer that prints a measurement result in response to a measurement completion signal or an instruction from an examiner or the like, an output portion that outputs the measurement result to an external memory, a server, or the like, and a voice output portion that notifies an operation status or the like. Note that the controller 30 may be provided inside the base 11, the stand 12, or the like.

Furthermore, the acquisition optical system 20 is coupled to the controller 30 via a cable (not illustrated) inside the main body portion 13. Then, the controller 30 controls and drives (including movement) the light source of the illumination optical system, the operation portion of the photographing optical system, and the like in the acquisition optical system 20. Furthermore, the controller 30 is coupled to the movement actuator 15a, the swing actuator 18a, the elevation actuator 19a, the display 25, the operation portion 26 including the control lever 16 (operation button 16a) and the software key, the storage 31, a stand position sensor 33, a swing sensor 34, an elevation sensor 35, and a distance sensor 36 inside the main body portion 13 via a cable (not illustrated). The controller 30 outputs a predetermined control command to the movement actuator 15a, the swing actuator 18a, the elevation actuator 19a, and the display 25 in accordance with an operation signal from the operation portion 26 to control them. Furthermore, in the ophthalmic device 10, power is supplied from a commercial power source to the controller 30, and the controller 30 supplies power to the coupled portions.

The stand position sensor 33 is a sensor that detects a position of the stand 12 (acquisition optical system 20) on the base 11 in the left-right direction and the front-rear direction and a position of the stand 12 (acquisition optical system 20) with respect to the base 11 in the up-down direction. The stand position sensor 33 outputs the detected position information of the stand 12 to the controller 30. The stand position sensor 33 of the first embodiment has a fixed sensor attached to the base 11 and a movement sensor attached to the stand 12 and moving together with the stand 12, and detects the position of the stand 12 in each of the left-right direction, the front-rear direction, and the up-down direction with respect to the base 11 based on the interval between the both sensors. Note that the stand position sensor 33 may be a contact type sensor or a non-contact type sensor.

The swing sensor 34 is a sensor that detects a rotation angle of the main body portion 13 (acquisition optical system 20) in the left-right direction. The swing sensor 34 outputs the detected angle information of the main body portion 13 to the controller 30. The swing sensor 34 of the first embodiment has a fixed sensor on the side of the stand 12 that supports the main body portion 13 and a movement sensor on the side of the main body portion 13 that moves together with the main body portion 13, and detects the rotation angle of the main body portion 13 in the left-right direction based on the interval between the both sensors. Note that the swing sensor 34 may be a contact sensor or a non-contact sensor.

The elevation sensor 35 is a sensor that detects a rotation angle of the main body portion 13 (acquisition optical system 20) in the up-down direction. The elevation sensor 35 outputs the detected angle information of the main body portion 13 to the controller 30. The elevation sensor 35 of the first embodiment has a fixed sensor on the side of the stand 12 that supports the main body portion 13 and a movement sensor on the side of the main body portion 13 that moves together with the main body portion 13, and detects the rotation angle of the main body portion 13 in the up-down direction based on the interval between the both sensors. Note that the elevation sensor 35 may be a contact sensor or a non-contact sensor.

The distance sensor 36 is a sensor that detects a distance from the main body portion 13 (acquisition optical system 20) to the eye E to be examined, that is, the interval therebetween. The distance sensor 36 outputs the detected distance information to the controller 30. The distance sensor 36 of the first embodiment detects a straight-line distance from the objective lens portion 21 which is a portion most protruding toward the subject side to the eye E to be examined, in the main body portion 13. The distance sensor 36 is provided, for example, on a surface of the main body portion 13 facing the subject. The distance sensor 36 of the first embodiment includes a light source such as an LED or an LD and a light receiving element therein, receives reflected light from a measurement target (for example, the face of the subject) of light emitted from the light source by the light receiving element, converts the reflected light into a distance, and outputs the distance. Note that the distance sensor 36 may be, for example, one using ultrasonic waves or infrared rays or a stereo camera as long as it detects the distance (interval) from the main body portion 13 to the eye E to be examined, may be a distance sensor having another configuration, and is not limited to the configuration of the first embodiment.

Hereinafter, display on the display 25 controlled by the controller 30 of the first embodiment will be described.

In the ophthalmic device 10 of the first embodiment, as illustrated in Fig. 3, a first display area 27a and a second display area 27b are set on the display screen 25a of the display 25 by the controller 30. Here, the controller 30 displays, in the first display area 27a, an image F of the eye fundus Ef of the eye E to be examined acquired by the acquisition optical system 20 to be displayed. In addition, the controller 30 displays, in the second display area 27b, positional relationship information J indicating a positional relationship between the eye E to be examined and the acquisition optical system 20 to be displayed. Note that the sizes and arrangement of the first display area 27a and the second display area 27b can be arbitrarily set, and for example, the controller 30 may set the second display area 27b on the entire surface of the display screen 25a. Furthermore, the controller 30 may display an arbitrary image or information other than the image F, a software key, or the like in the first display area 27a. Furthermore, the controller 30 may display an arbitrary image or information, software keys, and the like in an area other than the first display area 27a and the second display area 27b.

The "positional relationship information J" is information objectively representing an actual position of the acquisition optical system 20 with respect to an actual position of the eye E to be examined. The positional relationship information J is calculated by the controller 30 based on a distance from the eye E to be examined to the acquisition optical system 20 in the left-right direction, a distance from the eye E to be examined to the acquisition optical system 20 in the front-rear direction, a distance from the eye E to be examined to the acquisition optical system 20 in the up-down direction, a rotation angle of the acquisition optical system 20 in the left-right direction centered on the rotation axis 18b, a rotation angle of the acquisition optical system 20 in the up-down direction centered on the center axis 19b, and the like. The controller 30 obtains distance information and rotation angle information necessary for calculation of the positional relationship information J from detection results of the stand position sensor 33, the swing sensor 34, the elevation sensor 35, and the distance sensor 36. Furthermore, the controller 30 may obtain the positional relationship information J from operation amounts of the movement actuator 15a, the swing actuator 18a, and the elevation actuator 19a.

In the first embodiment, the positional relationship information J is displayed by the first graphic image 41 illustrated in Fig. 4. The first graphic image 41 has a first icon 41A indicating an eyeball model indicating the eye E to be examined and a second icon 41B indicating a device model indicating the ophthalmic device 10 including the acquisition optical system 20.

The first icon 41A and the second icon 41B are indicated by an isometric projection method. Here, the "isometric projection method" is a projection method in which an inclined three-dimensional object is drawn so that angles formed in three directions of front and rear, left and right, and up and down become equal angles (120 degrees) to each other. Therefore, the first graphic image 41 displayed by the isometric projection method is basically a perspective view of the eye E to be examined and the ophthalmic device 10 as viewed obliquely from above.

In addition, in the first embodiment, the first icon 41A is displayed by an eyeball model image indicated by an isometric projection method schematically indicating the eye E to be examined in which an outer shell of the eye E to be examined is divided in the left-right direction and an inside is hollow. In Fig. 4, a portion corresponding to a crystalline lens of the eye E to be examined is denoted by a reference sign α, and a portion corresponding to the eye fundus Ef of the eye E to be examined is denoted by a reference sign β. Furthermore, the first icon 41A is not limited to the icon illustrated in Fig. 4, and may be, for example, an eyeball model image schematically indicating an arbitrary part (for example, only an anterior eye portion, only a crystalline lens, and the like) of the eye E to be examined. In addition, the first icon 41A may be an eyeball model image schematically indicating the entire internal structure or the left half or the right half by displaying the outer shell of the eye E to be examined in a semi-transmissive manner, or may be an eyeball model image schematically indicating the internal structure by breaking a part (for example, an upper quarter) of the eye E to be examined. In the example illustrated in Figs. 3 and 4, a blood vessel image of the left half of the eye E to be examined is superimposed and displayed on the eyeball model, but the blood vessel image may not be displayed.

On the other hand, the second icon 41B is displayed as a device model image indicated by an isometric projection method schematically indicating the ophthalmic device 10 including the acquisition optical system 20, the position change mechanism 15, the control lever 16, and the swing mechanism 18 and the elevation mechanism 19 of the angle change mechanism 17. In Fig. 4, in the second icon 41B, a portion indicating the acquisition optical system 20 is denoted by a reference sign 101, a portion indicating the position change mechanism 15 is denoted by a reference sign 102, a portion indicating the control lever 16 is denoted by a reference sign 103, a portion indicating the swing mechanism 18 is denoted by a reference sign 104, and a portion indicating the elevation mechanism 19 is denoted by a reference sign 105. In addition, the device model image indicated as the second icon 41B may have a shape different from that of the actual ophthalmic device 10, or may be partially enlarged, reduced, or deformed so that the examiner can easily grasp the positional relationship information J. In the example illustrated in Fig. 4, the acquisition optical system 20 is schematically illustrated in a reduced size in order to improve visibility.

The scale of the second icon 41B with respect to the first icon 41A may be displayed at equal magnification, or may be enlarged or reduced at an arbitrary magnification without being at equal magnification. In the example illustrated in Fig. 4, the second icon 41B is displayed in a reduced size with respect to the first icon 41A, the first icon 41A imitating the eye E to be examined is displayed in a relatively enlarged size, and the second icon 41B imitating the ophthalmic device 10 is displayed in a relatively reduced size. Since the second icon 41B is displayed at a non-equal magnification with respect to the first icon 41A, the examiner can easily grasp the positional relationship information J.

Furthermore, the controller 30 may superimpose and display an image (hereinafter, referred to as a "crystalline lens opacity distribution image") indicating a state of the current opacity distribution of the crystalline lens on a portion α corresponding to the crystalline lens of the eye E to be examined in the first icon 41A. Detection of the crystalline lens opacity distribution is detected by using a known device (for example, see Shack-Hartmann Sensor, Japanese Patent No. 6775337, or the like). Furthermore, the crystalline lens opacity position is identified by a known identification method (for example, see JP 2019-170710 A, or the like, such as a method using optical coherence tomography (OCT) scanning).

In addition, the controller 30 may superimpose and display an image (hereinafter, referred to as a "retinal image") indicating a current state of the retina on a portion β corresponding to the eye fundus Ef of the eye E to be examined in the first icon 41A. The retina image is combined with the first icon 41A, which is an eyeball model image, based on a known technique (for example, see JP 2020-156622 A).

Note that the crystalline lens opacity distribution image and the retina image may be displayed (so-called lantern display or balloon display) in an arbitrary area that is within the second display area 27b and is not superimposed on the first icon 41A.

Furthermore, as illustrated in Fig. 4, the controller 30 superimposes a light flux image γ indicating the light flux between the eye E to be examined and the acquisition optical system 20 and an optical axis image δ indicating the optical axis O from the acquisition optical system 20 toward the eye E to be examined on the first graphic image 41 and displays the superimposed image as the positional relationship information J.

Note that the light flux image γ indicates how light travels between the eye E to be examined and the acquisition optical system 20. In general, in the eye fundus camera, ring illumination is used to remove reflection from the cornea or crystalline lens of the eye E to be examined. Therefore, the light flux of the photographing light passes through the pupil center of the eye E to be examined, but the light flux of the illumination light does not pass through the pupil center. The light flux image γ superimposed and displayed on the first graphic image 41 may indicate the light flux of the illumination light or the light flux of the photographing light.

In addition, the optical axis image δ indicates the orientation of the light output from the acquisition optical system 20. In the ophthalmic device 10 of the first embodiment, the optical axis image δ can be set by the examiner, and the controller 30 can perform alignment of the ophthalmic device 10 based on the set optical axis image δ.

That is, first, the examiner visually recognizes the first graphic image 41 displayed in the second display area 27b. At this time, when the crystalline lens opacity distribution image is superimposed and displayed on the portion α indicating the crystalline lens, the examiner can grasp the opacity distribution in the crystalline lens. Then, the examiner performs a touch operation in the portion α corresponding to the crystalline lens and designates a position of the optical axis O passing through the inside of the crystalline lens. Next, the examiner performs a touch operation in the portion β corresponding to the eye fundus Ef of the eye E to be examined, and designates a position of the eye fundus Ef to be observed. At this time, when the retina image is superimposed and displayed on the portion β corresponding to the eye fundus Ef, the examiner performs a touch operation on the retina image. Therefore, it is possible to designate a position to be observed while grasping the state of the retina of the eye fundus Ef. Subsequently, the controller 30 specifies the position where the touch operation is performed in the portion α corresponding to the crystalline lens and the position where the touch operation is performed in the portion β corresponding to the eye fundus Ef of the eye E to be examined. Then, the controller 30 displays a straight line connecting the touch position in the portion α corresponding to the crystalline lens and the touch position in the portion β indicating the eye fundus Ef as the optical axis image δ. As a result, the optical axis image δ is set by the examiner. Then, after the optical axis image δ is set, the controller 30 controls the position change mechanism 15 and the angle change mechanism 17 to change the position or the orientation of the acquisition optical system 20 so that the actual optical axis O of the acquisition optical system 20 coincides with the optical axis image δ set by the examiner, and performs alignment of the ophthalmic device 10.

In addition, the controller 30 can rotate the first graphic image 41 about a predetermined position set in the second display area 27b to display. In order to rotate the first graphic image 41, the examiner may perform a touch operation on the first graphic image 41, or the examiner may perform a touch operation on a software key (not illustrated) displayed at an arbitrary position on the display screen 25a. The software key for rotating the first graphic image 41 may be superimposed and displayed on the first graphic image 41. By rotating the first graphic image 41, the examiner can visually recognize the positional relationship between the eye E to be examined and the acquisition optical system 20 from a desired angle.

As illustrated in Figs. 5A to 5D, the controller 30 may display a first software key 42 and a second software key 43 in the second display area 27b in addition to the first graphic image 41 indicating the positional relationship information J. Furthermore, the controller 30 may display a vertical line (turning reference axis) L1 passing through a turning point G of the eyeball and the rotation axis 18b serving as a rotation center of the swing mechanism 18 to be superimposed on the first graphic image 41.

The first software key 42 is a software key that causes the controller 30 to output a control command to the angle change mechanism 17 when a touch operation is performed by the examiner. The controller 30 rotates the main body portion 13 (acquisition optical system 20) in the left-right direction or the up-down direction in response to the touch operation on the first software key 42.

In the example illustrated in Figs. 5A to 5D, the first software key 42 has a first arrow 42a, a second arrow 42b, a third arrow 42c, and a fourth arrow 42d. The first software key 42 is superimposed and displayed on the first graphic image 41. Then, when the examiner performs a touch operation on the first arrow 42a, the controller 30 rotates the main body portion 13 in the right direction. When the examiner performs a touch operation on the second arrow 42b, the controller 30 rotates the main body portion 13 in the left direction. When the examiner performs a touch operation on the third arrow 42c, the controller 30 rotates the main body portion 13 in the upward direction. When the examiner performs a touch operation on the fourth arrow 42d, the controller 30 rotates the main body portion 13 in the downward direction.

The second software key 43 is a software key that causes the controller 30 to output a control command to the position change mechanism 15 when a touch operation is performed by the examiner. In response to the touch operation on the second software key 43, the controller 30 moves the stand 12 (acquisition optical system 20) in any one of the front-rear direction, the left-right direction, and the up-down direction.

In the example illustrated in Figs. 5A to 5D, the second software key 43 has a fifth arrow 43a, a sixth arrow 43b, a seventh arrow 43c, an eighth arrow 43d, a ninth arrow 43e, and a tenth arrow 43f. The second software key 43 is superimposed and displayed on the first graphic image 41. Then, when the examiner performs a touch operation on the fifth arrow 43a, the controller 30 moves the stand 12 in the right direction. When the examiner performs a touch operation on the sixth arrow 43b, the controller 30 moves the stand 12 in the left direction. When the examiner performs a touch operation on the seventh arrow 43c, the controller 30 moves the stand 12 in the forward direction. When the examiner performs a touch operation on the eighth arrow 43d, the controller 30 moves the stand 12 in the rearward direction. When the examiner performs a touch operation on the ninth arrow 43e, the controller 30 moves the stand 12 in the upward direction. When the examiner performs a touch operation on the tenth arrow 43f, the controller 30 moves the stand 12 in the downward direction.

Note that the touch operation on the first software key 42 and the second software key 43 is not limited to a normal single tap operation, and may be a double tap operation, a long press operation (long tap), a flick operation in a predetermined direction, a swipe operation in a predetermined direction, a drag operation in a predetermined direction, a pinch-in operation, a pinch-out operation, or the like.

Furthermore, the controller 30 changes the control of the position change mechanism 15 or the angle change mechanism 17 according to a type, a number of times, an operation amount (tap time, drag amount, or the like), and the like of the touch operation on the first software key 42 or the second software key 43 displayed on the display 25. That is, a moving direction or a moving amount of the stand 12 by the position change mechanism 15, and a rotation direction and a rotation angle amount of the main body portion 13 by the angle change mechanism 17 are set according to the number of times the examiner taps the first arrow 42a, the operation amount, or the like.

Furthermore, when rotating the main body portion 13 in the left-right direction or the up-down direction, the controller 30 of the first embodiment changes the display of the second icon 41B of the first graphic image 41 following the actual change in the rotation angle of the main body portion 13 as illustrated in Fig. 5B. When the position of the stand 12 is changed, the controller 30 changes the display of the second icon 41B of the first graphic image 41 following the change in the actual position of the stand 12 as illustrated in Fig. 5C or 5D. As a result, the ophthalmic device 10 of the first embodiment can display the positional relationship between the eye E to be examined and the acquisition optical system 20 in real time.

In addition, here, a vertical line (turning reference axis) L1 passing through the turning point G of the eyeball and the rotation axis 18b serving as a rotation center of the swing mechanism 18 are superimposed and displayed on the first graphic image 41. Therefore, the examiner can easily recognize a degree of positional deviation, a direction of deviation, and the like between the eye E to be examined and the acquisition optical system 20 based on the deviation between the vertical line (turning reference axis) L1 and the rotation axis 18b.

As illustrated in Figs. 5B, 5C, and 5D, the controller 30 also changes the display position of the first software key 42 or the second software key 43 following the display change of the second icon 41B. Therefore, it is possible to suppress a decrease in operability of the first software key 42 or the second software key 43.

Hereinafter, the operation of the ophthalmic device 10 of the first embodiment will be described.

The ophthalmic device 10 of the first embodiment includes an acquisition optical system 20, a position change mechanism 15 and an angle change mechanism 17, a display 25, and a controller 30. Here, the acquisition optical system 20 acquires eye information of an eye E to be examined. In addition, the position change mechanism 15 has an electric movement actuator 15a, and changes the respective positions of the acquisition optical system 20 in the left-right direction, the front-rear direction, and the up-down direction by using the power of the movement actuator 15a. The angle change mechanism 17 has an electric swing actuator 18a and an elevation actuator 19a. The angle change mechanism 17 changes a rotation angle (orientation) of the acquisition optical system 20 in the left-right direction by using the power of the swing actuator 18a, and changes a rotation angle (orientation) of the acquisition optical system 20 in the up-down direction by using the driving force of the elevation actuator 19a. In addition, the display 25 has a display screen 25a that can be visually recognized by an examiner who is an operator who operates the position change mechanism 15 and the angle change mechanism 17. Then, the controller 30 displays positional relationship information J indicating a positional relationship between the eye E to be examined and the acquisition optical system 20 on the display screen 25a of the display 25.

Therefore, in the ophthalmic device 10 of the first embodiment, the positional relationship in which the eye E to be examined and the acquisition optical system 20 are disposed can be objectively grasped by the examiner who is the operator based on the positional relationship information J displayed on the display 25. Then, by objectively grasping the positional relationship between the eye E to be examined and the acquisition optical system 20, the examiner can accurately recognize the positional relationship between the eye E to be examined and the acquisition optical system 20, and improve the operability when controlling the position or the rotation angle (orientation) of the acquisition optical system 20.

Furthermore, in the ophthalmic device 10 of the first embodiment, the controller 30 displays the positional relationship information J by the first graphic image 41 in which the first icon 41A representing the eyeball model indicating the eye E to be examined and the second icon 41B representing the device model indicating the ophthalmic device 10 including the acquisition optical system 20 are indicated by an isometric projection method.

As a result, the ophthalmic device 10 of the first embodiment can cause the examiner to grasp the positional relationship between the eye E to be examined and the ophthalmic device 10 including the acquisition optical system 20 when viewed from above. Therefore, the examiner can easily recognize the outline of the positional relationship between the eye E to be examined and the acquisition optical system 20.

Furthermore, in the ophthalmic device 10 of the first embodiment, the controller 30 displays, on the display 25, the light flux image γ indicating the light flux between the eye E to be examined and the acquisition optical system 20 as the positional relationship information J. Therefore, the ophthalmic device 10 of the first embodiment can cause the examiner to objectively grasp the progress of light between the eye E to be examined and the acquisition optical system 20. As a result, the examiner can control the position or the rotation angle of the acquisition optical system 20 after objectively recognizing the light flux between the eye E to be examined and the acquisition optical system 20, and the operation accuracy can be improved.

Furthermore, in the ophthalmic device 10 of the first embodiment, the controller 30 displays, on the display 25, the optical axis image δ indicating the optical axis O of the acquisition optical system 20 as the positional relationship information J. Therefore, the ophthalmic device 10 of the first embodiment can cause the examiner to objectively grasp the orientation of the optical axis O from the acquisition optical system 20 toward the eye E to be examined. As a result, the examiner can control the position or the rotation angle of the acquisition optical system 20 after objectively recognizing the position where the optical axis O of the acquisition optical system 20 passes on the eye E to be examined, and the operation accuracy can be improved.

In particular, in the first embodiment, both the light flux image γ and the optical axis image δ are displayed to be superimposed on the first graphic image 41. Therefore, the state of the light flux between the eye E to be examined and the acquisition optical system 20 and the orientation of the optical axis O from the acquisition optical system 20 can be easily understood by the examiner.

Note that the light flux image γ and the optical axis image δ are not necessarily superimposed and displayed on the first graphic image 41. That is, since the ophthalmic device 10 only needs to cause the examiner to grasp the status of the light flux and the orientation of the optical axis O, for example, as illustrated in Fig. 6, the eye E to be examined and the acquisition optical system 20 may be indicated by a geometric figure such as a circle, a quadrangle, a triangle, or an ellipse. In Fig. 6, the eye E to be examined is represented by a circle 200, and the acquisition optical system 20 is represented by a square 201. Furthermore, also in Fig. 6, a portion corresponding to the crystalline lens of the eye E to be examined is denoted by a reference sign α, and a portion corresponding to the eye fundus Ef of the eye E to be examined is denoted by a reference sign β.

In the ophthalmic device 10 of the first embodiment, the display 25 has a touch panel function. Then, the controller 30 displays the first software key 42 on the display 25, and rotates the main body portion 13 (acquisition optical system 20) by the angle change mechanism 17 when a touch operation is performed on the first software key 42.

As a result, the examiner can change the orientation of the acquisition optical system 20 by performing the touch operation on the first software key 42 displayed on the display 25. Therefore, operability at the time of changing the orientation of the acquisition optical system 20 can be improved.

In particular, in the first embodiment, the first software key 42 is superimposed on the first graphic image 41 and displayed. Therefore, in the ophthalmic device 10 of the first embodiment, the examiner can easily grasp how the acquisition optical system 20 moves when the touch operation is performed on the first software key 42. Moreover, the display of the first graphic image 41 and the first software key 42 is changed following the actual movement of the main body portion 13. That is, the device model image (second icon 41B) displayed as the first graphic image 41 moves in the same manner as the actual main body portion 13. This makes it easy to grasp the result of the touch operation on the first software key 42.

Further, in the ophthalmic device 10 of the first embodiment, the controller 30 displays the second software key 43 on the display 25 having a touch panel function, and when the touch operation is performed on the second software key 43, moves the stand 12 (acquisition optical system 20) with the position change mechanism 15.

As a result, the examiner can change the position of the acquisition optical system 20 by performing the touch operation on the second software key 43 displayed on the display 25. Therefore, operability at the time of changing the position of the acquisition optical system 20 can be improved.

Moreover, in the first embodiment, the second software key 43 is superimposed and displayed on the first graphic image 41. Therefore, in the ophthalmic device 10 of the first embodiment, the examiner can easily grasp how the acquisition optical system 20 moves by performing the touch operation on the second software key 43. Moreover, the display of the first graphic image 41 and the second software key 43 is changed following the actual movement of the main body portion 13. That is, the device model image (second icon 41B) displayed as the first graphic image 41 moves in the same manner as the actual stand 12. This also makes it easy to grasp the result of the touch operation on the second software key 43.

Note that the controller 30 does not necessarily have to superimpose and display the first software key 42 or the second software key 43 on the first graphic image 41. The first software key 42 or the second software key 43 may be independently displayed at an arbitrary position on the display screen 25a of the display 25 having a touch panel function.

Further, the controller 30 may use the first graphic image 41 as the first software key 42 or the second software key 43. In this case, when a touch operation is performed on the first graphic image 41, the controller 30 changes the position of the stand 12 or changes the rotation angle of the main body portion 13 by the position change mechanism 15 or the angle change mechanism 17. As a result, it is possible to make the examiner feel the sensation of directly moving the ophthalmic device 10, and it is possible to further easily grasp the movement of the acquisition optical system 20.

In the ophthalmic device 10 of the first embodiment, the touch operation on the first software key 42 and the second software key 43 displayed on the display 25 is at least one of a normal tap operation (single tap), a double tap operation, a long press operation (long tap), a flick operation, a swipe operation, a drag operation, a pinch-in operation, and a pinch-out operation. Therefore, a touch operation considering operability by the examiner can be assigned to the first software key 42 and the like, and operability can be improved.

Furthermore, the controller 30 changes the control of the position change mechanism 15 or the angle change mechanism 17 according to a type, a number of times, an operation amount (tap time, drag amount, or the like), and the like of the touch operation on the first software key 42 or the second software key 43 displayed on the display 25. Therefore, the examiner can intuitively control the position change mechanism 15 or the angle change mechanism 17, and operability can be improved.

Furthermore, in the ophthalmic device 10 of the first embodiment, the optical axis image δ can be set by the examiner, and the controller 30 performs alignment of the acquisition optical system 20 based on the set optical axis image δ. That is, the controller 30 displays, on the display 25, the optical axis image δ indicating the optical axis O from the acquisition optical system 20, and changes the position or the rotation angle of the acquisition optical system 20 according to the touch operation on the optical axis image δ.

As a result, the orientation of the optical axis O can be set based on the crystalline lens opacity distribution, for example, and the eye fundus Ef can be observed while avoiding a portion where the crystalline lens is opacified due to the influence of cataract or the like.

Furthermore, in the ophthalmic device 10 of the first embodiment, a crystalline lens opacity distribution image may be superimposed and displayed on the portion α corresponding to the crystalline lens of the eye E to be examined in the first icon 41A. In this case, the examiner can set the optical axis image δ by performing a touch operation on the crystalline lens opacity distribution image. As a result, the controller 30 can change at least one of the position or the orientation of the acquisition optical system 20 by the position change mechanism 15 and the angle change mechanism 17 according to the touch operation on the crystalline lens opacity distribution image. As a result, the examiner can control the position or the orientation of the acquisition optical system 20 so that the eye fundus image can be acquired while accurately avoiding the opaque area of the crystalline lens of the eye E to be examined.

In addition, in the ophthalmic device 10 of the first embodiment, the retinal image may be superimposed and displayed on the portion β corresponding to the eye fundus Ef of the eye E to be examined in the first icon 41A. In this case, the examiner can set the optical axis image δ by performing a touch operation on the retinal image. As a result, the controller 30 can change at least one of the position or the orientation of the acquisition optical system 20 by the position change mechanism 15 and the angle change mechanism 17 according to the touch operation on the retinal image. As a result, the examiner can accurately observe and photograph a desired position of the eye fundus Ef of the eye E to be examined according to the state of the retina, and can appropriately acquire desired eye information (eye fundus image).

Note that the image superimposed on the first graphic image 41 and referred to when setting the optical axis image δ may be an image other than a crystalline lens opacity distribution image or a retina image. For example, as the image superimposed on the portion α corresponding to the crystalline lens, a transillumination image or a B-scan line image of an OCT image may be used. In addition, as the image to be superimposed on the portion β corresponding to the retina, a photographed image of the retina by an infrared camera, a retina scan image by OCT, or a retina projection image by OCT may be used. In a case where the B-scan image of the OCT image is used as the retina image, it is possible to grasp the status of the target image in real time.

Furthermore, in the ophthalmic device 10 of the first embodiment, an example is illustrated in which the light flux image γ indicating the light flux between the eye E to be examined and the acquisition optical system 20 is displayed as the positional relationship information J. Here, the examiner may perform a touch operation on the light flux image γ to arbitrarily change the orientation or the position of the light flux image γ. Then, the controller 30 may control the position change mechanism 15 and the angle change mechanism 17 such that the actual light flux coincides with the light flux image γ changed by the touch operation, change the position or the orientation of the acquisition optical system 20, and perform alignment of the ophthalmic device 10. That is, the controller 30 may change at least one of the position or the orientation of the acquisition optical system 20 by the position change mechanism 15 and the angle change mechanism 17 according to the touch operation on the light flux image γ.

Even in this case, the examiner can accurately designate a desired position where observation and photographing in the eye E to be examined are desired, and can appropriately acquire desired eye information (eye fundus image).

In the ophthalmic device 10 of the first embodiment, the display 25 is provided in the main body portion 13 accommodating the acquisition optical system 20. That is, the display 25 is disposed in an environment in which the examiner can directly confirm the positional relationship between the eye E to be examined and the acquisition optical system 20. As a result, the examiner who has visually recognized the positional relationship information J displayed on the display 25 can operate the position change mechanism 15 or the angle change mechanism 17 while directly comparing the displayed positional relationship information J with the actual positional relationship between the eye E to be examined and the acquisition optical system 20. Therefore, a more accurate operation can be performed.

Further, in the ophthalmic device 10 of the first embodiment, the controller 30, the movement actuator 15a of the position change mechanism 15, the swing actuator 18a and the elevation actuator 19a of the angle change mechanism 17, and the display 25 are coupled to each other inside the main body portion 13 via a cable (not illustrated). Therefore, as compared with a case where the controller 30 or the like is coupled via a wireless communication network such as wide area wireless communication or near field wireless communication, the transmission/reception speed of various signals is faster, and the transmission/reception of signals can be stably performed.

### (Second Embodiment)

In the ophthalmic device 10 of the first embodiment, an example in which the positional relationship information J is displayed by the first graphic image 41 has been described, but the positional relationship information J may be indicated by other display. That is, in an ophthalmic device 10 of the second embodiment, as illustrated in Fig. 7, the positional relationship information J is displayed by a second graphic image 44. Since the configuration of the ophthalmic device 10 of the second embodiment is similar to that of the ophthalmic device 10 of the first embodiment, the description thereof will be omitted.

The second graphic image 44 is an image in which a third icon 44C indicating an eyeball model indicating an eye E to be examined and a fourth icon 44D indicating a device model indicating the ophthalmic device 10 including the acquisition optical system 20 are indicated by a third trigonometry. Here, the "third trigonometry" is a method of drawing a three-dimensional object placed at a third angle by a front view projected on a front vertical plane, a plan view projected on an upper horizontal plane, a left side view projected on a left vertical plane, and the like.

That is, the third icon 44C includes a plan view, a right side view, and a front view of an eyeball model schematically illustrating the eye E to be examined in which the outer shell of the eye E to be examined is divided in the left-right direction and the inside is hollow. The fourth icon 44D includes a plan view, a right side view, and a front view of a device model schematically illustrating the ophthalmic device 10 including the acquisition optical system 20, the position change mechanism 15, the control lever 16, and the swing mechanism 18 and the elevation mechanism 19 of the angle change mechanism 17. Therefore, the second graphic image 44 displayed by the third trigonometry is a plan view of the eye E to be examined and the ophthalmic device 10 as viewed from above, a right side view as viewed from the right side, or a front view as viewed from the examiner side. In Fig. 7, in the fourth icon 44D, a portion indicating the acquisition optical system 20 is denoted by a reference sign 101, a portion indicating the position change mechanism 15 is denoted by a reference sign 102, a portion indicating the control lever 16 is denoted by a reference sign 103, a portion indicating the swing mechanism 18 is denoted by a reference sign 104, and a portion indicating the elevation mechanism 19 is denoted by a reference sign 105.

As described above, in the ophthalmic device 10 of the second embodiment, the positional relationship information J is displayed by the second graphic image 44 in which the third icon 44C and the fourth icon 44D are indicated by the third trigonometry. Therefore, the positional relationship between the eye E to be examined and the acquisition optical system 20 such as a distance from the acquisition optical system 20 to the eye E to be examined and a rotation angle of the acquisition optical system 20 can be grasped in detail by the examiner who visually recognizes the display 25.

In the second graphic image 44 illustrated in Fig. 7, the eyeball model indicating the eye E to be examined and the device model indicating the ophthalmic device 10 are displayed in three views of a front view, a plan view, and a side view, but are not necessarily displayed in three views. The third icon 44C and the fourth icon 44D of the second graphic image 44 are images indicating the eyeball model and the device model by the third trigonometry, but may be displayed by one or more of a front view, a plan view, a left side view, and a right side view. That is, the second graphic image 44 may include, for example, only the third icon 44C indicating the eyeball model indicating the eye E to be examined in a plan view and the fourth icon 44D indicating the device model indicating the ophthalmic device 10 in a plan view.

In addition, the controller 30 may superimpose and display a crystalline lens opacity distribution image on a portion corresponding to the crystalline lens in the third icon 44C, or superimpose and display a retina image on a portion corresponding to the retina in the third icon 44C. In addition, the controller 30 may superimpose and display the light flux image γ or the optical axis image δ on the second graphic image 44.

Further, as illustrated in Fig. 8, in addition to the second graphic image 44 indicating the positional relationship information J, the controller 30 may display the first software key 42 that rotates the acquisition optical system 20 by the touch operation and the second software key 43 that moves the acquisition optical system 20 by the touch operation in the second display area 27b.

As in the first embodiment, the first software key 42 has a first arrow 42a, a second arrow 42b, a third arrow 42c, and a fourth arrow 42d. The second software key 43 has a fifth arrow 43a, a sixth arrow 43b, a seventh arrow 43c, an eighth arrow 43d, a ninth arrow 43e, and a tenth arrow 43f.

In addition, as illustrated in Fig. 9, the controller 30 may display a first scale image 45a and a second scale image 45b imitating scales indicating the rotation angle of the acquisition optical system 20 with respect to the eye E to be examined as the positional relationship information J. Here, the first scale image 45a indicates a rotation angle of the acquisition optical system 20 in the left-right direction. The second scale image 45b indicates a rotation angle of acquisition optical system 20 in the up-down direction.

Note that, in the example illustrated in Fig. 9, the first scale image 45a and the second scale image 45b are scales indicating a rotation angle around the pupil center position of the eye E to be examined, and are set to 0 degrees in a state where the eye E to be examined and the acquisition optical system 20 face each other (a state where the optical axis O is orthogonal to the eye fundus Ef). In the example illustrated in Fig. 9, the first scale image 45a and the second scale image 45b are displayed in an overlapping manner on the second graphic image 44.

Furthermore, as illustrated in Fig. 9, the controller 30 may display a first numerical value image 46a and a second numerical value image 46b indicating numerical values indicating the rotation angle of the acquisition optical system 20 with respect to the eye E to be examined as the positional relationship information J. Here, the first numerical value image 46a indicates a rotation angle of the acquisition optical system 20 in the left-right direction. The second numerical value image 46b indicates a rotation angle of the acquisition optical system 20 in the up-down direction.

In the example illustrated in Fig. 9, the first numerical value image 46a and the second numerical value image 46b are set to 0 degrees in a state where the eye E to be examined and the acquisition optical system 20 face each other (a state where the optical axis O is orthogonal to the eye fundus Ef).

In this manner, by displaying the positional relationship information J by the first scale image 45a and the second scale image 45b, or the first numerical value image 46a and the second numerical value image 46b, the examiner can more accurately grasp the distance from the acquisition optical system 20 to the eye E to be examined or the rotation angle of the acquisition optical system 20.

Furthermore, in the ophthalmic device 10 of the second embodiment, when a touch operation is performed on the first scale image 45a and the second scale image 45b, or the first numerical value image 46a and the second numerical value image 46b, the controller 30 outputs a control command to the angle change mechanism 17 in response to the touch operation on the first scale image 45a. Then, the controller 30 may rotate the main body portion 13 (acquisition optical system 20) in the left-right direction or the up-down direction by the angle change mechanism 17.

That is, the controller 30 may control the angle change mechanism 17 by using the first scale image 45a and the second scale image 45b, or the first numerical value image 46a and the second numerical value image 46b as software keys to rotate the main body portion 13 (acquisition optical system 20) in the left-right direction or the up-down direction.

As a result, the angle change mechanism 17 can be controlled by a touch operation without displaying a software key (for example, the first software key 42, the second software key 43, and the like) for controlling the angle change mechanism 17 on the display 25. As a result, it is possible to suppress an excessive display content of the display 25 and to improve operability.

Although the ophthalmic device of the present invention has been described based on the first embodiment and the second embodiment, the specific configuration is not limited to these embodiments, and modifications, additions, and the like of design are allowed without departing from the gist of the invention according to each claim of the claims.

In the ophthalmic devices 10 of the first and second embodiments, an example in which the first software key 42 and the second software key 43 are superimposed and displayed on the positional relationship information J (the first graphic image 41 and the second graphic image 44) has been described. However, the present invention is not limited thereto.

For example, as illustrated in Fig. 10A, the controller 30 sets a third display area 27c at a position different from the second display area 27b for displaying the first graphic image 41, on the display screen 25a of the display 25. Then, as illustrated in Fig. 10B, the controller 30 displays a control lever image 50, a first software key 42, and a second software key 43 in the third display area 27c.

The control lever image 50 is an image indicating a control lever model schematically indicating the control lever 16 by an isometric projection method. The display of the control lever image 50 may be changed by tilting or rotating the control lever model according to the operation of the first software key 42 or the second software key 43.

The first software key 42 is a software key that causes the controller 30 to output a control command to the angle change mechanism 17 when a touch operation is performed by the examiner. As illustrated in Fig. 10B, the first software key 42 has a first arrow 42a, a second arrow 42b, a third arrow 42c, and a fourth arrow 42d.

The second software key 43 is a software key that causes the controller 30 to output a control command to the position change mechanism 15 when a touch operation is performed by the examiner. As illustrated in Fig. 10B, the second software key 43 has a fifth arrow 43a, a sixth arrow 43b, a seventh arrow 43c, an eighth arrow 43d, a ninth arrow 43e, and a tenth arrow 43f.

As described above, by displaying the first software key 42 and the second software key 43 in the third display area 27c set separately from the second display area 27b indicating the positional relationship information J, it is possible to display only the positional relationship information J in the second display area 27b while enabling control of the angle change mechanism 17 and the position change mechanism 15 by a touch operation on the display 25. Consequently, the excessive display content in the second display area 27b can be suppressed, and the positional relationship information J can easily be visually recognized.

In addition, by displaying the first software key 42 and the second software key 43 together with the control lever image 50, the examiner can easily image the movement of the control lever 16, and can easily intuitively grasp the operation of the acquisition optical system 20. As a result, the examiner can more accurately recognize the positional relationship between the eye E to be examined and the acquisition optical system 20.

Note that, in the example illustrated in Figs. 10A and 10B, the control lever image 50 is an image in which the control lever model is indicated by an isometric projection method, but the present invention is not limited thereto. For example, as in a control lever image 51 of a first modification illustrated in Fig. 11, it may be an image in which the control lever model is indicated by a circular or triangular geometric figure. Note that in a case where the control lever model is indicated by a circular geometric figure, it is possible to indicate a state in which the ophthalmic device 10 is viewed in a plan view. On the other hand, when the control lever model is indicated by a triangular geometric figure, the measurement direction can be indicated by, for example, the orientation of a vertex. In the example illustrated in Fig. 11, the position of the eye E to be examined is indicated as the position facing the vertex of the most acute angle.

In addition, the controller 30 may display a plurality of control lever images 51 and display the first software key 42 and the second software key 43 in a distributed manner (see Fig. 11). Further, the controller 30 may display only the first software key 42 or the second software key 43 configured by a plurality of arrows or the like in the third display area 27c without displaying the control lever image.

In addition, in the ophthalmic device 10 of the first embodiment, an example in which the crystalline lens opacity distribution image and the retina image may be displayed to be superimposed on the first graphic image 41 has been described. In addition, an example of displaying the image (light flux image γ) indicating the current state of the light flux between the eye E to be examined and the acquisition optical system 20 and the image (optical axis image δ) indicating the current state of the optical axis O of the acquisition optical system 20 has been described. Here, the crystalline lens opacity image, the retina image, the light flux image γ, and the optical axis image δ are site information of the eye E to be examined indicating a state of a part of the eye E to be examined. Furthermore, in the ophthalmic device 10 of the second embodiment, an example in which the current rotation angle of the acquisition optical system 20 with respect to the eye E to be examined is displayed has been described. However, the various types of information displayed on the display 25 by the controller 30 are not limited thereto.

That is, the controller 30 may display, on the display 25, for example, an image indicating a current state of an anterior eye portion of the eye E to be examined, an image indicating a current state of a posterior eye portion of the eye E to be examined, an image indicating a current state of a vitreous body of the eye E to be examined, and the like as the site information of the eye E to be examined. Furthermore, the controller 30 may display a current distance from the eye E to be examined to the acquisition optical system 20.

Furthermore, the controller 30 may display not only the current state of the crystalline lens opacity distribution, the retinal area, and the like but also a target state of the site information of the eye E to be examined on the display 25. That is, the controller 30 may display the target state of the anterior eye portion of the eye E to be examined, the target state of the posterior eye portion of the eye E to be examined, and the target states of various sites such as the vitreous body, the crystalline lens, and the retinal area of the eye E to be examined on the display screen 25a of the display 25. In addition, the controller 30 may display a target rotation angle of the acquisition optical system 20 with respect to the eye E to be examined and a target distance from the eye E to be examined to the acquisition optical system 20.

Then, the controller 30 may enlarge or reduce the site information of the eye E to be examined displayed on the display 25 or change the center position of the display in response to a touch operation by the examiner, an operation of the operation portion 26, or the like, and displays, on the display 25, the site information again. That is, for example, when the examiner performs a pinch-out operation on a position of a papilla in the eye fundus image of the eye E to be examined, the display may be switched to an enlarged image of the eye fundus image centered on the position of the touched papilla, or when the examiner performs a pinch-in operation on the position of the papilla in the eye fundus image of the eye E to be examined, the display may be switched to a reduced image of the eye fundus image centered on the position of the touched papilla. Furthermore, when the examiner performs a single tap operation on the position of the papilla in the eye fundus image of the eye E to be examined, the eye fundus image may be changed to an eye fundus image centered on the position of the touched papilla and displayed.

In this way, by changing an enlargement ratio and a center position of the displayed information according to the examiner's operation on the site information of the eye E to be examined, the site of the eye E to be examined that the examiner wants to confirm in detail and the site of the eye E to be examined that the examiner wants to know the outline can be displayed in a state desired by the examiner as necessary.

In addition, in the ophthalmic device 10 of the first embodiment, an example in which the controller 30, the position change mechanism 15 and the angle change mechanism 17, and the display 25 are coupled via a cable (not illustrated) inside the main body portion 13 has been described. However, the present invention is not limited thereto. The controller 30, the position change mechanism 15 and the angle change mechanism 17, and the display 25 may be coupled via wide area wireless communication such as a mobile phone network. In this case, remote control can be performed, and the eye information of the eye E to be examined can be acquired even when the examiner is not near the subject. Note that as an example of a wide area wireless communication standard using a mobile phone network, there is Long Term Evolution (LTE), for example.

Furthermore, the controller 30, the position change mechanism 15 and the angle change mechanism 17, and the display 25 may be coupled via near field wireless communication. In this case, although the wireless communication is performed, the communication speed is faster than that of the wide area wireless communication, and occurrence of a time lag in transmission and reception of various signals can be suppressed. Note that examples of near field wireless communication standards include Bluetooth (registered trademark) Low Energy (BLE) communication, Wi-Fi (registered trademark), and the like. In addition, although it is difficult to clearly distinguish between the wide area wireless communication and the near field wireless communication, here, the wide area wireless communication is performed when the communication distance is 100 m or more (generally several km), and the near field wireless communication is performed when the communication distance is less than 100 m.

Furthermore, the controller 30, the position change mechanism 15 and the angle change mechanism 17, and the display 25 may be coupled via a management server. In this case, various signals transmitted and received between the controller 30, the position change mechanism 15 and the angle change mechanism 17, and the display 25 can be collectively managed by the management server. As a result, it is also possible to collectively manage the plurality of ophthalmic devices 10.

In the first embodiment, the display 25 is provided in the main body portion 13 accommodating the acquisition optical system 20, and the display 25 is disposed in an environment in which the examiner can directly confirm the positional relationship between the eye E to be examined and the acquisition optical system 20. However, the present invention is not limited thereto. For example, the display 25 and the controller 30 may be configured by a portable information terminal, a laptop computer, or the like that can be separated from the main body portion 13, and may be disposed in an environment (remote place) in which the positional relationship between the eye E to be examined and the acquisition optical system 20 cannot be directly confirmed by the examiner who is the operator. In this case, the examiner who has visually recognized the positional relationship information J displayed on the display 25 can operate the position change mechanism 15 or the angle change mechanism 17 from a remote place or issue an instruction to the subject or the like in the vicinity of the acquisition optical system 20 based on the displayed positional relationship information J.

In the ophthalmic device 10 of the first embodiment, an example in which the position change mechanism 15 or the angle change mechanism 17 is operated by the examiner has been described, but the present invention is not limited thereto. For example, the subject may be an operator and operate the position change mechanism 15 and the angle change mechanism 17 by performing a touch operation or the like on the display 25 by himself/herself.

The operations of the position change mechanism 15 and the angle change mechanism 17 are not limited to the touch operation on the display 25 having a touch panel function, and the position change mechanism 15 and the like may be operated by tilting or rotating the control lever 16 which is the operation portion 26.

In the ophthalmic device 10 of the first embodiment, an example in which the first graphic image 41, the light flux image γ, and the optical axis image δ are displayed as the positional relationship information J has been described. In the ophthalmic device 10 of the second embodiment, an example in which the second graphic image 44, the first scale image 45a and the second scale image 45b, and the first numerical value image 46a and the second numerical value image 46b are displayed as the positional relationship information J has been described. Here, at least one of the display modes indicating the positional relationship information J may be displayed, and for example, can be arbitrarily selected and displayed, such as displaying the first graphic image 41, and the first scale image 45a and the second scale image 45b together.

In addition, when the positional relationship information J is displayed by using the graphic image, not only a graphic image indicated by an isometric projection method or a third trigonometry but also a graphic image indicated by a perspective projection method or an oblique projection method, for example, may be used.

Furthermore, for example, an arbitrary image may be selectively switched and displayed, such as switching and displaying the first graphic image 41 and the second graphic image 44. Note that selection and switching of the display are performed by displaying a selection menu on the display 25 and allowing the examiner to make a selection by a touch operation, or allowing the examiner to make a selection by operating the operation portion 26 such as a keyboard.

In addition, in the ophthalmic device 10 of the first embodiment, an example has been described in which the rotation axis 18b is set to coincide with the pupil center position of the eye E to be examined by alignment, and a swinging operation and an elevation operation of the acquisition optical system 20 are performed with the pupil center position as the rotation center. However, as long as the swing operation and the elevation operation of the acquisition optical system 20 are performed with the rotation axis 18b and the center axis 19b as the rotation center, the position where the rotation axis 18b and the center axis 19b coincide with each other in the eye E to be examined may be appropriately set, and the present invention is not limited to the configuration of each embodiment. For example, it is conceivable that the rotation axis 18b is set to coincide with a turning point or a corneal vertex of the eye E to be examined, and in such a configuration, the swing operation and the elevation operation of the acquisition optical system 20 can be performed with the turning point or the corneal vertex as the rotation center.

In addition, an example has been described in which the ophthalmic device 10 of the first embodiment has the position change mechanism 15 and the angle change mechanism 17 as the optical system change mechanism, and the acquisition optical system 20 can be moved in each of the left-right direction, the front-rear direction, and the up-down direction with respect to the eye E to be examined, and can be rotated in the left-right direction and the up-down direction. However, the present invention is not limited thereto. The ophthalmic device to which the present invention is applied may be, for example, an ophthalmic device having only the position change mechanism 15 as the optical system change mechanism or an ophthalmic device having only the angle change mechanism 17 as the optical system change mechanism. Alternatively, an electric ophthalmic device may be used in which either the position change mechanism 15 or the angle change mechanism 17 is electrically operated, and the other is driven by manual operation.

In addition, an example in which the ophthalmic device 10 of the first embodiment is an eye fundus camera having the acquisition optical system 20 that acquires an eye fundus image as eye information of the eye E to be examined has been described. However, the present invention is not limited thereto, and can be applied to any ophthalmic device having an acquisition optical system movable with respect to the eye E to be examined. For example, the ophthalmic device may be an autokeratorefractometer or an autokeratometer that measures a refractive power (visual acuity) of the eyeball and the cornea of the eye E to be examined as the eye information, a tonometer that measures an intraocular pressure of the eye E to be examined as the eye information, or the like.

### CROSS-REFERENCE TO RELATED APPLICATIONS

The present application claims priority based on Japanese Patent Application No. 2021-126964 filed with the Japan Patent Office on August 2, 2021, the entire disclosure of which is entirely incorporated herein by reference.

## Claims

1. An ophthalmic device comprising:
an acquisition optical system that acquires eye information of an eye to be examined;
an electric optical system change mechanism that changes at least one of a position or an orientation of the acquisition optical system with respect to the eye to be examined;
a display that is visually recognizable by an operator who operates the optical system change mechanism; and
a controller that displays, on the display, positional relationship information indicating a positional relationship between the eye to be examined and the acquisition optical system.

2. The ophthalmic device according to claim 1, wherein
the positional relationship information is displayed by at least one of a first graphic image in which a first icon indicating the eye to be examined and a second icon indicating the acquisition optical system are indicated by an isometric projection method, a second graphic image in which a third icon indicating the eye to be examined and a fourth icon indicating the acquisition optical system are indicated by a third trigonometry, a light flux image indicating a light flux between the eye to be examined and the acquisition optical system, an optical axis image indicating an optical axis of the acquisition optical system, a scale image imitating a scale indicating at least one of a distance from the eye to be examined to the acquisition optical system or a rotation angle of the acquisition optical system with respect to the eye to be examined, and a numerical value image indicating at least one of a distance from the eye to be examined to the acquisition optical system or a rotation angle of the acquisition optical system with respect to the eye to be examined as a numerical value.

3. The ophthalmic device according to claim 1 or 2, wherein
the display has a touch panel function,
the optical system change mechanism has an angle change mechanism that rotates the acquisition optical system in at least one of a left-right direction or an up-down direction about a reference axis set in advance, and
the controller that displays, on the display, a first software key, and rotates the acquisition optical system by the angle change mechanism when a touch operation is performed on the first software key.

4. The ophthalmic device according to claim 1 or 2, wherein
the display has a touch panel function,
the optical system change mechanism has a position change mechanism that moves the acquisition optical system in at least one of a left-right direction, a front-rear direction, and an up-down direction with respect to the eye to be examined, and
the controller that displays, on the display, a second software key, and moves the position change mechanism by the acquisition optical system when a touch operation is performed on the second software key.

5. The ophthalmic device according to claim 3, wherein
the touch operation is at least one of a single tap operation, a double tap operation, a long press operation, a flick operation, a swipe operation, a drag operation, a pinch-in operation, and a pinch-out operation.

6. The ophthalmic device according to claim 5, wherein
the controller changes control of the optical system change mechanism according to at least one of a type, a number of times, and an operation amount of the touch operation.

7. The ophthalmic device according to claim 1 or 2, wherein
the display has a touch panel function, and
the controller displays, on the display, an optical axis image indicating an optical axis of the acquisition optical system, and changes at least one of a position or an orientation of the acquisition optical system by the optical system change mechanism according to a touch operation on the optical axis image.

8. The ophthalmic device according to claim 1 or 2, wherein
the display has a touch panel function, and
the controller displays, on the display, a retina image indicating a retina of the eye to be examined, and changes at least one of a position or an orientation of the acquisition optical system by the optical system change mechanism according to a touch operation on the retina image.

9. The ophthalmic device according to claim 1 or 2, wherein
the display has a touch panel function, and
the controller displays, on the display, a light flux image indicating a light flux between the eye to be examined and the acquisition optical system, and changes at least one of a position or an orientation of the acquisition optical system by the optical system change mechanism according to a touch operation on the light flux image.

10. The ophthalmic device according to claim 1 or 2, wherein
the display has a touch panel function, and
the controller displays, on the display, a scale image imitating a scale indicating at least one of a distance from the eye to be examined to the acquisition optical system or a rotation angle of the acquisition optical system with respect to the eye to be examined, and changes at least one of a position or an orientation of the acquisition optical system by the optical system change mechanism according to a touch operation on the scale image.

11. The ophthalmic device according to claim 1 or 2, wherein
the display has a touch panel function, and
the controller that displays, on the display, a numerical value image indicating at least one of a distance from the eye to be examined to the acquisition optical system or a rotation angle of the acquisition optical system with respect to the eye to be examined as a numerical value, and changes at least one of a position or an orientation of the acquisition optical system by the optical system change mechanism according to a touch operation on the numerical value image.

12. The ophthalmic device according to claim 1 or 2, wherein
the display is installed in any one of an environment in which the operator is capable of directly confirming the positional relationship or an environment in which the operator is not capable of directly confirming the positional relationship.

13. The ophthalmic device according to claim 1 or 2, wherein
the controller, the optical system change mechanism, and the display are coupled via a cable, coupled via wide area wireless communication, coupled via near field wireless communication, or coupled via a management server.

14. The ophthalmic device according to claim 1 or 2, wherein
the controller displays, on the display, a current state or a target state of at least one of site information of the eye to be examined including at least one of an anterior eye portion of the eye to be examined, a posterior eye portion of the eye to be examined, a vitreous body of the eye to be examined, a crystalline lens of the eye to be examined, and a retinal area of the eye to be examined, a light flux between the eye to be examined and the acquisition optical system, an optical axis of the acquisition optical system, a distance from the eye to be examined to the acquisition optical system, and an orientation of the acquisition optical system with respect to the eye to be examined.

15. The ophthalmic device according to claim 14, wherein
the controller enlarges or reduces the site information indicated on the display, or changes a display center position, and displays, on the display, to display the site information again.

16. The ophthalmic device according to claim 4, wherein
the touch operation is at least one of a single tap operation, a double tap operation, a long press operation, a flick operation, a swipe operation, a drag operation, a pinch-in operation, and a pinch-out operation.

17. The ophthalmic device according to claim 16, wherein
the controller changes control of the optical system change mechanism according to at least one of a type, a number of times, and an operation amount of the touch operation.
